# FASCICULE DE BREVET EUROPEEN

(11) **EP 1 737 529 B1**
(45) Date de publication et mention de la délivrance du brevet: **11.07.2007**
(21) Numéro de dépôt: 05717632.3
(22) Date de dépôt: 14.02.2005
(51) Int. Cl.: A61M 25/06

(54) **DISPOSITIF POUR L'INTRODUCTION D'UN CATHETER AVEC CAGE DE SECURITE ANTI-PIQUE A LAME FLEXIBLE**
VORRICHTUNG ZUM EINFÜHREN EINES KATHETERS MIT NICHT STECHENDEM SICHERHEITSKÄFIG MIT FLEXIBLER KLINGE
DEVICE FOR INTRODUCING A CATHETER WITH A SECURITY NON-PIERCING CAGE PROVIDED WITH A FLEXIBLE BLADE

(30) Priorité: 02.03.2004 FR 0402126
(43) Date de publication de la demande: 03.01.2007
(73) Titulaire: VYGON, F-95440 Ecouen (FR)
(72) Inventeur: CARREZ, Jean-Luc, F-95440 Ecouen (FR); DALLE, Valéry, F-60270 Gouvieux (FR); GUYOMARC'H, Pierrick, F-95120 Ermont (FR); HUET, Jean-Max, F-92110 Clichy (FR)
(74) Mandataire: Texier, Christian
(86) Numéro de dépôt international: PCT/FR2005/000343
(87) Numéro de publication internationale: WO 2005/094939

(56) Documents cités:
- EP-A- 0 554 841
- EP-A- 0 753 317
- FR-A- 2 836 385
- US-A- 5 322 517
- US-A- 5 447 501

## Description

L'invention vise à éviter les risques d'une piqûre accidentelle au retrait d'une aiguille de ponction utilisée pour l'introduction d'un cathéter dans un site du corps au travers de la peau.

De très nombreux dispositifs anti-pique ont été proposés à cette fin.

La publication FR 2 836 385 décrit un dispositif dans lequel l'aiguille toute entière avec son embase est piégée dans un étui après son retrait.

Les publications EP 0 554 841 et US 5 322 517 décrivent des moyens de sécurité qui comprennent une cage pour piéger la pointe de l'aiguille après son retrait, cette cage contenant une lame en acier à ressort qui présente une branche traversée par l'aiguille, et une autre branche qui est précontrainte par l'aiguille dans une position inactive où elle porte latéralement contre l'aiguille et qui vient en position active devant l'aiguille lorsque ce contact est supprimé du fait d'un retrait de l'aiguille.

La publication EP 0 753 317 décrit une cage qui coulisse sur l'aiguille et qui comporte une lame en acier à ressort précontrainte par contact avec l'aiguille dans une position inactive tant que l'aiguille traverse la cage et qui est libérée et agit pour dévier l'aiguille quand l'aiguille est rentrée dans la cage.

La publication US 5 447 501 décrit un dispositif qui comprend un ressort qui est précontraint par l'aiguille dans une position inactive et qui dévie l'aiguille lorsqu'il est libéré par le retrait de l'aiguille.

D'autres dispositifs à cage sont encore décrits dans les publications EP 0 456 694 (ou US 5 322 517), US 623 499, US 5 176 655, et EP 0 891 198 (ou US 6 001 080).

Un but de la présente invention est de fournir un dispositif simple, à cage et à lame flexible, de fonctionnement automatique et sûr, dans lequel la lame n'est pas précontrainte par l'aiguille.

L'invention concerne un dispositif pour l'introduction d'un cathéter dans un site du corps, notamment une veine, au travers de la peau, ce cathéter étant équipé d'une embase proximale, ce dispositif comprenant une aiguille qui présente une extrémité de ponction et comprenant une cage anti-pique qui prolonge l'embase du cathéter en direction proximale, cette cage déterminant une chambre traversée à coulisse par l'aiguille d'une entrée proximale à une sortie distale et munie d'une lame flexible en acier à ressort pour retenir dans la chambre l'extrémité de ponction de l'aiguille lorsque l'aiguille est retirée de la canule, cette lame étant disposée dans le travers de la chambre à proximité de l'entrée proximale de la chambre perpendiculairement à l'aiguille et traversée par l'aiguille, la lame et l'aiguille comprenant des moyens coopérant pour que la lame soit au repos et traversée librement par l'aiguille lorsque l'aiguille est poussée en direction distale et pour que la lame arrête l'aiguille et soit fléchie par l'aiguille lorsque l'aiguille est tirée en direction proximale au-delà d'une position axiale déterminée, en sorte que la lame fléchie incline l'aiguille et exerce sur l'aiguille inclinée une force de rappel qui tend à repousser l'aiguille en direction distale jusqu'à butée de l'extrémité de ponction de l'aiguille inclinée contre une paroi de la chambre.

Dans une réalisation préférée, la lame flexible présente une perforation pour le passage de l'aiguille et l'aiguille présente localement une section d'aiguille modifiée apte à être arrêtée par la perforation de la lame lors du mouvement de retrait de l'aiguille, cette section modifiée étant située à une distance de l'extrémité de ponction de l'aiguille telle que le contact de la section modifiée avec la perforation de la lame se produise après que cette extrémité soit rentrée dans la chambre lors du mouvement de retrait de l'aiguille

Dans des modes de réalisation préférés, l'invention présente encore une plusieurs des caractéristiques suivantes:
- la chambre présente une paroi de fond en direction distale qui forme un sillon dans lequel vient se planter l'extrémité de ponction de l'aiguille inclinée ;
- la cage présente en avant de la chambre un nez qui s'emboîte sans coincement dans l'embase du cathéter et qui est traversé longitudinalement par une lumière pour le passage de l'aiguille;
- l'embase du cathéter présente un rebord externe et la cage porte un organe qui présente un bec qui vient en prise avec ce rebord pour la fixation détachable de la cage à l'embase;
- le bec constitue une extrémité d'un levier et la lame flexible est conformée pour actionner par contact ce levier de façon à dégager le bec du rebord de l'embase du cathéter lorsque la lame est suffisamment fléchie.

On décrira ci-après différentes réalisations d'un dispositif conforme à l'invention pour la mise en place d'un cathéter court dans une veine, en référence aux dessins annexés sur lesquels :
- la figure 1 montre en coupe longitudinale une première réalisation prête à l'emploi, la lame flexible étant au repos ;
- la figure 2 montre la réalisation de la figure 1 au cours de deux phases successives de la manipulation de retrait de l'aiguille ;
- la figure 3 montre l'ensemble de la figure 1 après séparation de la cage et de la canule;
- la figure 4 est une vue agrandie d'un détail de l'ensemble de la figure 2 ;
- la figure 5 montre en coupe longitudinale une variante de réalisation de l'ensemble de la figure 1, prête à l'emploi, la lame flexible étant au repos ;
- la figure 6 montre la réalisation de la figure 5 au cours de phases successives de la manipulation d'extraction de l'aiguille ;
- la figure 7 est une vue agrandie d'un détail de l'ensemble de la figure 6;
- la figure 8 est une vue agrandie du même détail au cours d'une phase ultérieure de la manipulation d'extraction ;
- la figure 9 montre en coupe longitudinale une autre réalisation d'un ensemble selon l'invention prêt à l'emploi, la lame flexible étant au repos;

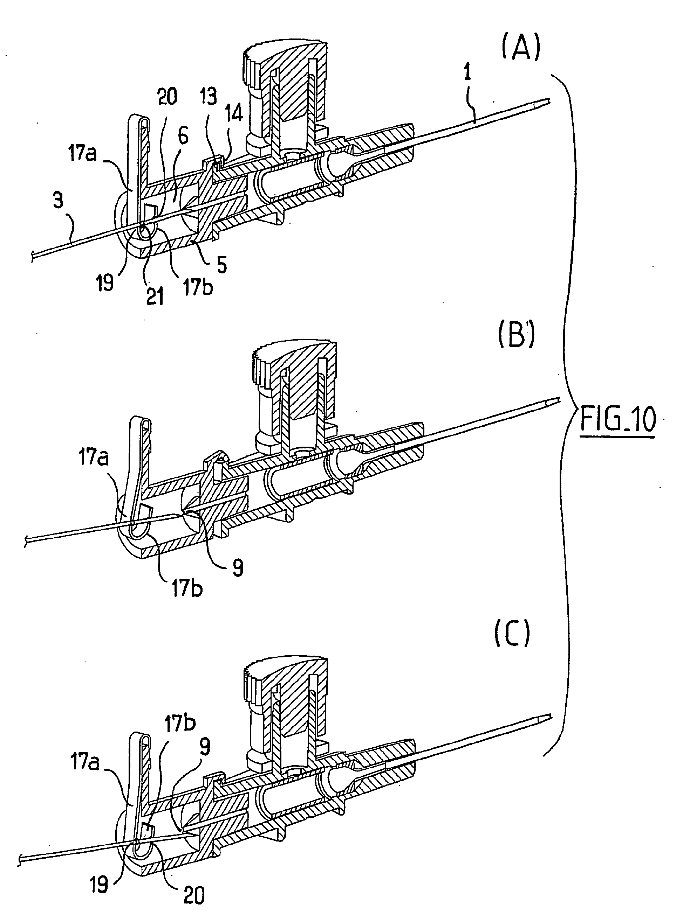

- la figure 10A est une perspective d'un ensemble selon la figure 9, dans laquelle l'embase de l'aiguille a été omise, dans laquelle l'ensemble a été coupé en deux par un plan de symétrie longitudinal et dans laquelle l'aiguille a été retirée jusqu'à ce que la pointe de l'aiguille soit sur le point de déboucher dans la chambre de la cage;
- la figure 10B est analogue à la figure 10A au cours d'une phase de retrait ultérieure, la pointe de l'aiguille étant arrivée dans la chambre de la cage, et l'aiguille provoquant une flexion de la lame flexible ;
- la figure 10C est analogue à la figure 10B au cours d'une phase ultérieure où la lame flexible a été repoussée par l'aiguille jusqu'à ce que la pointe de l'aiguille vienne buter contre la paroi frontale de la chambre de la cage ;
- la figure 11 montre l'ensemble de la figure 9 après séparation de la cage et de la canule;
- la figure 12 est une vue agrandie d'un détail de l'ensemble de la figure 9;
- la figure 13 montre en coupe longitudinale une autre réalisation d'un ensemble selon l'invention prêt à l'emploi, la lame flexible étant au repos;
- la figure 14 montre la réalisation de la figure 13 au cours de phases successives de la manipulation d'extraction de l'aiguille ;
- la figure 15 montre la réalisation de la figure 13 après séparation de la cage et de la canule, et
- la figure 16 est une vue agrandie d'un détail de l'ensemble de la figure 12.

Sur les figures, on a représenté une canule constituée d'un cathéter tubulaire court (1) muni d'une embase proximale (2), une aiguille (3) qui présente une extrémité de ponction (3a) et qui est munie d'une embase proximale (4), et une cage anti-pique

La cage (5) détermine une chambre (6) qui présente une entrée d'aiguille proximale (7) tournée vers l'embase de l'aiguille et une paroi de fond opposée (8) qui présente une sortie d'aiguille distale (9) tournée vers l'embase du cathéter.

De préférence, la paroi de fond de la chambre détermine autour de la sortie de la chambre un sillon (10).

La cage comporte en avant de la chambre un nez (11) qui s'emboîte sans coincement dans l'embase du cathéter et qui est traversé longitudinalement par une lumière (12) pour le passage de l'aiguille.

L'embase du cathéter présente un rebord externe (13), par exemple constitué par l'un des filets de l'embase lorsque celle-ci est filetée extérieurement, et la cage porte un bec mobile(14) qui vient en prise avec ce rebord pour retenir de façon détachable la cage sur l'embase.

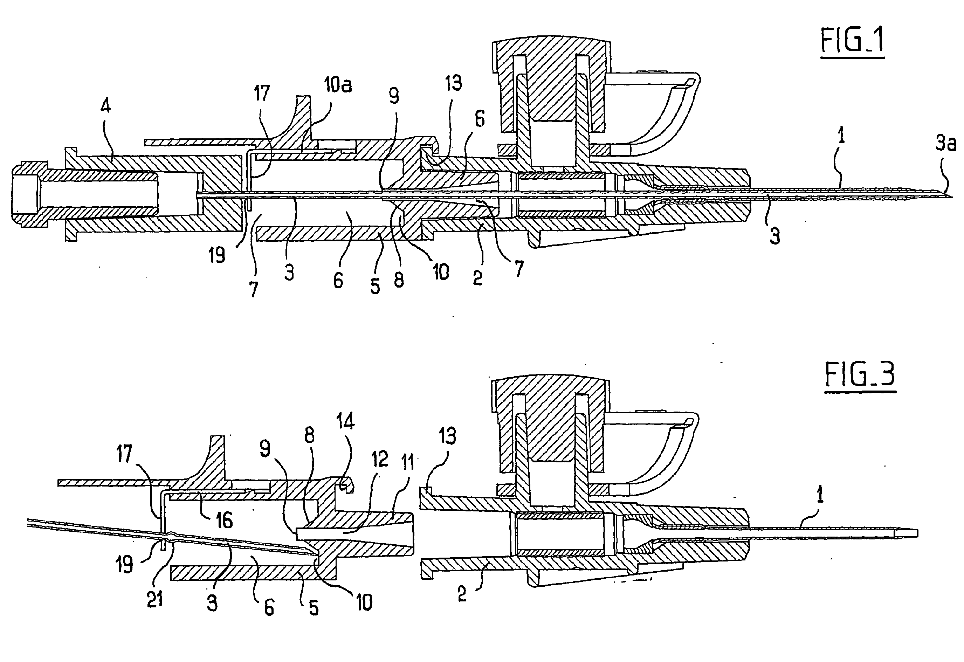

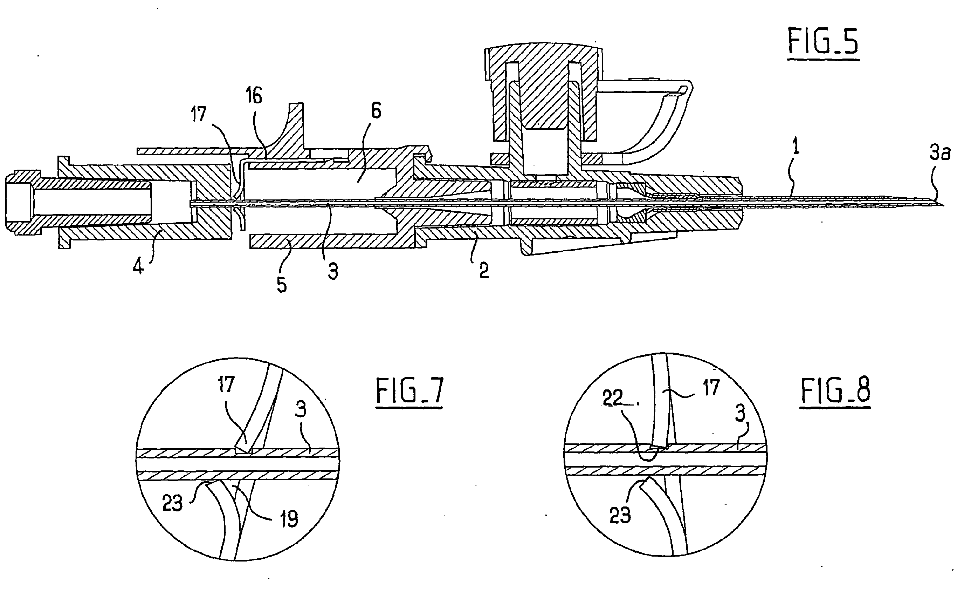

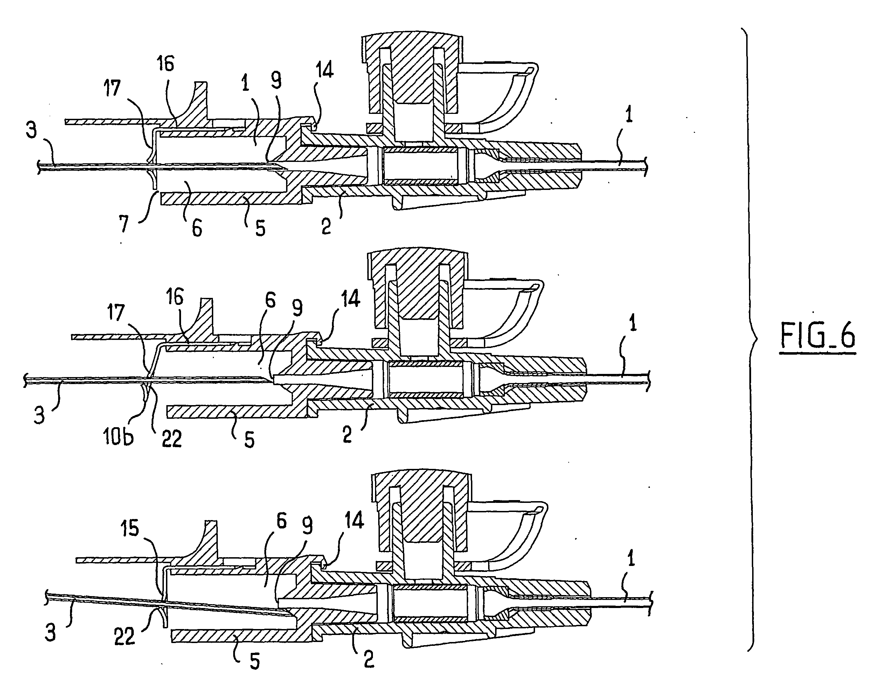

Dans les réalisations des figures 1 et 5, la cage comporte une lame en acier à ressort conformée en L qui présente une branche longitudinale (16) fixée à une paroi longitudinale de la chambre et une branche transversale flexible (17) située à proximité de l'entrée proximale (7) de la chambre et pourvue d'une perforation (19) axée sur la sortie (9) de la chambre lorsque cette branche flexible est au repos (figures 1 et 5) pour le passage de l'aiguille.

L'aiguille présente localement, de façon en soi connue, une modification de section choisie pour ne pas compromettre le coulissement de l'aiguille dans la canule tout en étant susceptible d'être arrêtée par la perforation (19) de la branche flexible de la lame qui se trouve à l'entrée de la chambre.

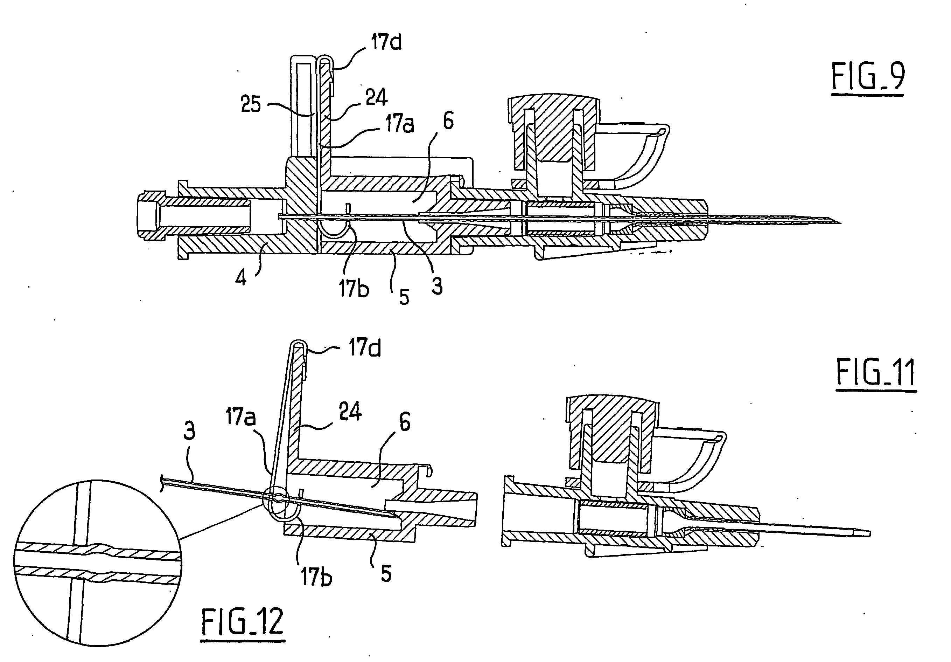

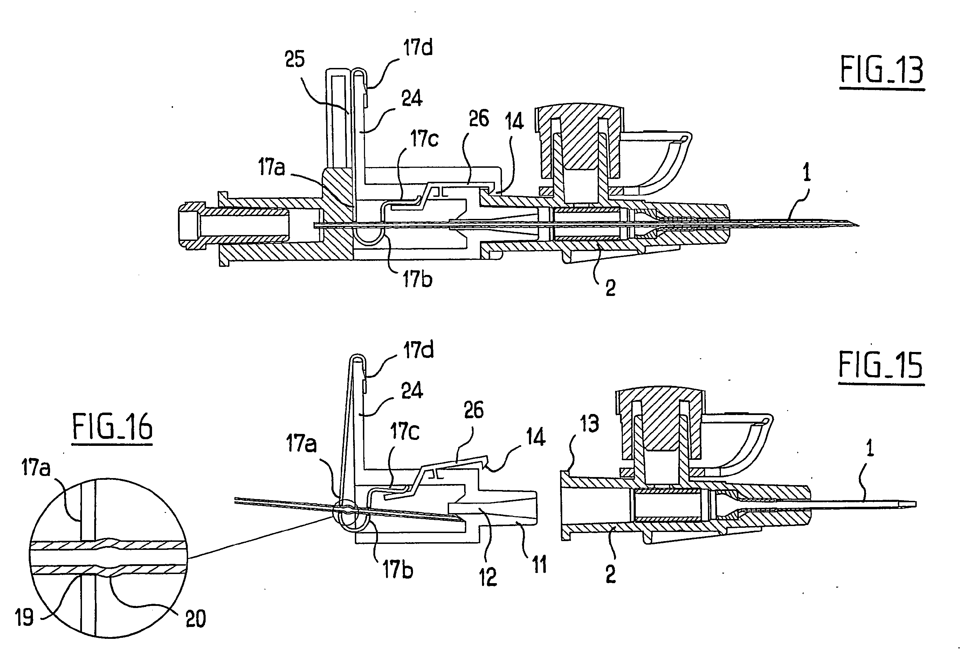

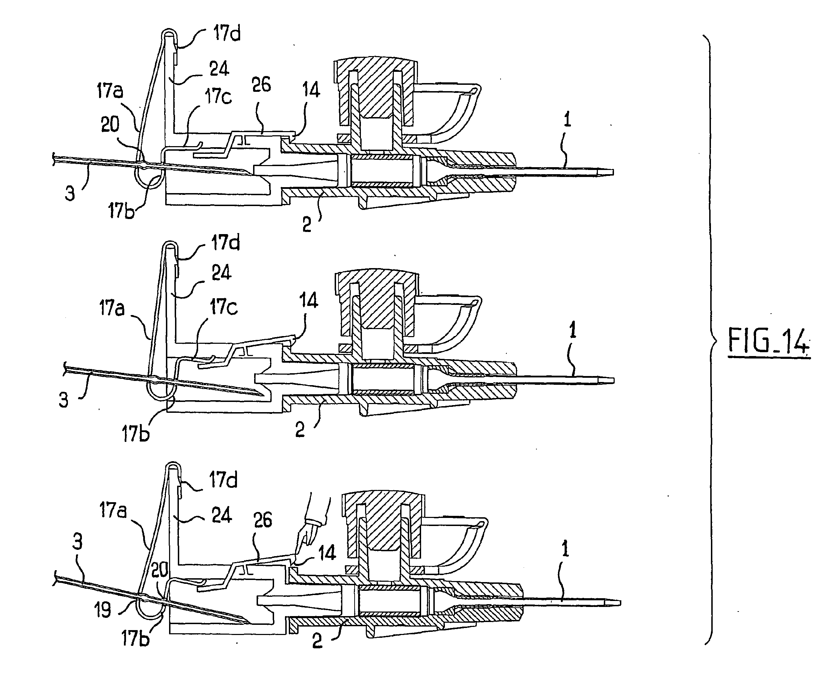

Dans les réalisations des figures 9 et 13, la branche flexible (17) est conformée en U, constituant une branche transversale arrière (17a) située à l'entrée de la chambre et pourvue d'une perforation (19) pour le passage de l'aiguille et une branche transversale avant (17b) parallèle à la première branche, située dans ladite chambre et pourvue d'une perforation (20) pour le passage de l'aiguille, et suffisamment large pour laisser passer également ladite section modifiée de l'aiguille, alors que la perforation (19) arrête cette section modifiée. Les perforations (19) et (20) sont alignées et axées sur la sortie de la chambre lorsque la branche flexible est au repos.

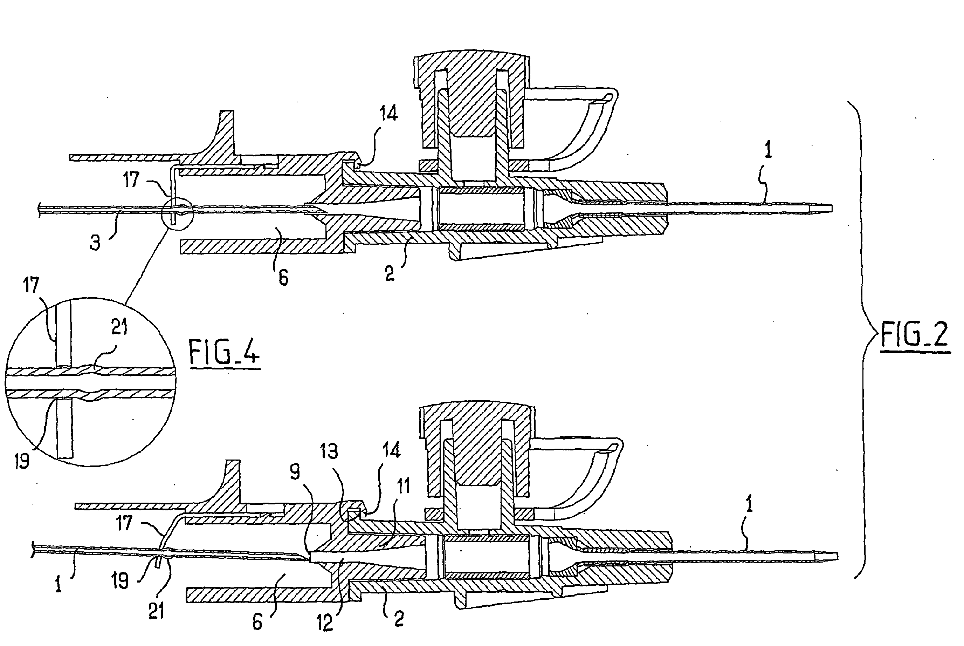

On a représenté, à titre indicatif et non limitatif, deux exemples en soi connus d'une telle modification, à savoir respectivement, une modification constituée par un renflement local (21) de la paroi de l'aiguille (fig.4) et une modification constituée par une fente (22) dans cette paroi (figs.7 et 8). Dans le premier cas, la perforation (19) de la lame flexible peut être banalement cylindrique tandis que dans le second cas, la lame présente à l'endroit de la perforation des griffes (23) susceptibles de mordre dans la paroi de l'aiguille.

On notera que dans le premier cas, l'aiguille ne sera pas bloquée dans la lame et pourra toujours coulisser dans le sens distal (vers l'avant) tandis que dans le second cas l'aiguille sera bloquée dans la lame.

Dans tous les cas, la modification de l'aiguille sera réalisée après enfilage de l'aiguille dans la lame.

Cette lame en U guide l'aiguille en deux points et l'oblige à prendre l'orientation imposée par la flexion de la lame.

Dans les réalisations des figures 9 et 13, la cage comporte un plateau transversal (24) en saillie latérale et contre lequel vient s'appuyer une paroi (25) de l'embase (4) de l'aiguille lorsque l'aiguille est en position de service.

Dans les réalisations des figures 9 et 13, la lame flexible (17b) est suspendue par une branche recourbée (17d) au plateau transversal (24) de la cage.

Dans la réalisation de la figure 13, pour permettre la séparation de la cage de l'embase du cathéter, le bec (14) qui assure la fixation provisoire de la cage sur l'embase constitue une extrémité d'un levier (26) et la lame flexible est conformée pour actionner par contact ce levier de façon à dégager le bec du rebord de l'embase du cathéter lorsque la lame a suffisamment fléchie. Dans le cas représenté à titre d'exemple uniquement, la lame flexible comporte à cet effet une troisième branche (17c), qui continue la deuxième branche sensiblement à l'équerre de cette branche et qui appuie sur ce levier pour l'actionner lorsque la lame fléchit.

On utilise le dispositif de la figure 1 comme suit:

Après avoir réalisé la ponction veineuse avec le dispositif tel que représenté sur la figure 1, on pousse le cathéter vers l'avant dans la veine en maintenant l'aiguille, la cage restant accrochée à l'embase du cathéter et s'éloignant de l'embase de l'aiguille.

Lorsque le cathéter est en place, on tire en arrière l'aiguille tout en maintenant le cathéter (fig.2), jusqu'à ce que le renflement de l'aiguille vienne au contact du trou de la lame qu'il ne peut traverser.

En continuant la traction arrière sur l'aiguille, on fléchit élastiquement la lame et le biseau de l'aiguille rentre dans la chambre. La déformation de la lame amène son trou à se désaxer et par-là désaxe l'aiguille qui s'incline dans la chambre. En continuant la traction arrière, on désolidarise la cage de l'embase du cathéter (fig.3).

La lame flexible reprend alors sa position de repos et repousse l'aiguille par l'intermédiaire du renflement. Le biseau désaxé vient se planter dans le sillon créé autour de la sortie de la chambre, il est alors bloqué.

Dans la variante de la figure 5, en tirant l'aiguille en arrière, on amène la fente de la paroi de l'aiguille au niveau des griffes de la lame. Les griffes rentrent dans celle-ci et assurent un blocage axial de l'aiguille. En poursuivant le mouvement de retrait de l'aiguille, on déforme la lame, la cage se sépare de la canule, la lame revient à sa position d'origine. Même si le biseau arrivait à se recentrer dans le trou, le biseau serait bloqué dans la cage. Un utilisateur qui voudrait réengager le biseau dans la sortie distale de la chambre ne le pourrait pas.

La réalisation de la figure 9 est utilisée comme les réalisations précédentes et bénéficie d'une sécurité renforcée du fait que l'aiguille est guidée par les deux perforations de la lame flexible en U qui coopèrent pour la contraindre à s'incliner lorsque la lame est fléchie.

Dans la réalisation de la figure 13, la traction sur l'aiguille, bloquée en recul arrière dans la chambre, amène à une traction arrière sur la lame. Quand l'extrémité de ponction de l'aiguille est dans la chambre, le bec de retenue peut monter sur la collerette de l'embase pour s'échapper vers l'arrière, ce qui permet de séparer la cage de l'embase.

L'invention n'est pas limitée à ces exemples de réalisation.

## Revendications

1. Dispositif pour l'introduction dans le corps, au travers de la peau., d'un cathéter (1) à embase proximale (2), ce dispositif comprenant une aiguille (3) qui présente une extrémité de ponction et une cage (5) qui prolonge l'embase en direction proximale, cette cage déterminant une chambre (6) traversée à coulisse par l'aiguille d'une entrée proximale (7) à une sortie distale opposée (9) et munie d'une lame flexible en acier à ressort (17 ;17b) pour retenir dans la chambre l'extrémité de ponction de l'aiguille lorsque l'aiguille est retirée de la canule, **caractérisé en ce que** cette lame est disposée dans le travers de la chambre à proximité de l'entrée proximale (7) de la chambre perpendiculairement à l'aiguille et traversée par l'aiguille, la lame et l'aiguille comprenant des moyens (19 ; 21 ; 22) coopérant pour que la lame soit au repos et traversée librement par l'aiguille lorsque l'aiguille est poussée en direction distale et pour que la lame arrête l'aiguille et soit fléchie par l'aiguille lorsque l'aiguille est tirée en direction proximale au-delà d'une position axiale déterminée, en sorte que la lame fléchie incline l'aiguille et exerce sur l'aiguille inclinée une force de rappel qui tend à repousser l'aiguille en direction distale jusqu'à butée de l'extrémité de ponction contre une paroi de la chambre.

2. Dispositif selon la revendication 1 dans lequel la lame flexible présente une perforation (19) pour le passage de l'aiguille et l'aiguille présente localement en avant de ladite perforation une section d'aiguille modifiée (21; 22) en sorte que cette section soit arrêtée par la perforation de la lame lors du mouvement de retrait de l'aiguille en direction proximale, cette section modifiée étant située à une distance de ponction de l'aiguille telle que le contact de la section modifiée avec la perforation de la lame se produise après que cette extrémité soit arrivée dans la chambre lors du mouvement de retrait de l'aiguille.

3. Dispositif selon la revendication 1 ou 2 dans lequel la chambre présente une paroi de fond (8) en direction distale qui comporte un sillon (10) dans lequel vient se planter l'extrémité de ponction (3a) de l'aiguille inclinée.

4. Dispositif selon l'une des revendications 1 à 3 dans lequel la lame flexible constitue une branche (17) d'une lame conformée en L et qui présente une branche longitudinale (16) fixée à une paroi longitudinale de la chambre et une branche transversale (17) qui constitue la lame flexible pourvue d'une perforation (19) pour le passage de l'aiguille.

5. Dispositif selon l'une des revendications 1 à 3 dans lequel la lame flexible est conformée en U, constituant une première branche transversale arrière (17a) située à l'entrée de la chambre et pourvue d'une perforation (19) pour le passage de l'aiguille et une deuxième branche transversale avant (17b) parallèle à la première branche, située dans ladite chambre et pourvue d'une perforation (20) pour le passage de l'aiguille, la perforation (19) de la branche arrière n'étant pas apte à laisser passer la section modifiée de l'aiguille mais la perforation (20) de la branche avant étant apte à laisser passer cette section.

6. Dispositif selon la revendication 5 dans lequel l'embase du cathéter présente un rebord externe (13) et la cage porte un levier mobile (26) qui présente un bec d'arrêt (14) en prise avec ce rebord dans une position du levier, et dans lequel la deuxième branche (17b) de la lame flexible se continue par une troisième branche (17c) sensiblement à l'équerre de la deuxième branche et qui actionne ledit levier pour supprimer la prise.

7. Dispositif selon l'une des revendications 1 à 6, dans lequel la cage comporte en avant de la chambre un nez (11) qui s'emboîte dans l'embase du cathéter et qui est traversé longitudinalement par une lumière (12) pour le passage de l'aiguille.

8. Dispositif selon l'une des revendications 1 à 7 dans lequel l'aiguille (3) est munie d'une embase (4) et dans lequel la cage comporte un plateau transversal (24) en saillie latérale contre lequel vient s'appuyer une paroi (25) de l'embase de l'aiguille lorsque l'aiguille est en position de service.

9. Dispositif selon la revendication 8 dans lequel ladite lame (12) est suspendue audit plateau (24) de la cage.

10. Dispositif selon l'une des revendications 1 à 9 dans lequel l'aiguille (3) comporte une embase (4) en appui contre la lame flexible lorsque l'aiguille est en position de service.

## Claims

1. A device for the insertion into the body, through the skin, of a catheter (1) with a proximal base (2), where this device comprising a needle (3) which has a puncture end and a cage (5), which extends the base in the proximal direction, where this chamber forms a chamber (6) through which the needle slides from a proximal entrance (7) to an opposite distal exit (9) and is equipped with a sprung flexible steel blade (17; 17b) to hold the puncture end of the needle in the chamber when the needle is withdrawn from the cannula, **characterised in that** this blade is positioned across the chamber close to the proximal entrance (7) of the chamber perpendicular to the needle and traversed by the needle, where the blade and the needle include means (19; 21; 22) that combine so that the blade is at rest and traversed freely by the needle when the needle is pushed in the distal direction, and so that the blade stops the needle and is bent by the needle when the needle is drawn in the proximal direction beyond a given axial position, so that the bent blade inclines the needle and applies a restoring force to the needle which tends to force the needle back in the distal direction until the puncture end comes up against a wall of the chamber.

2. A device according to claim 1, in which the flexible blade has a perforation (19) for the passage of the needle, and ahead of the said perforation, the needle has its section modified locally (21; 22) so that this section is stopped by the perforation in the blade during the withdrawal movement of the needle in the proximal direction, this modified section being located at a distance from the puncture end of the needle so that the contact of the modified section with the perforation in the blade occurs after this end has arrived in the chamber during the operation for removal of the needle.

3. A device according to claim 1 or 2, in which the chamber has an end wall (8) in the distal direction which includes a groove (10) into which the puncture end (3a) of the inclined needle enters.

4. A device according to any one of claims 1 to 3, in which the flexible blade constitutes a branch (17) of a blade shaped as an L, and which has a longitudinal branch (16) fixed to a longitudinal wall of the chamber and a transverse branch (17) which constitutes the flexible blade equipped with a perforation (19) for the passage of the needle.

5. A device according to any one of claims 1 to 3, in which the flexible blade has a U shape, constituting a first rear transverse branch (17a) located at the entrance of the chamber, and equipped with a perforation (19) for the passage of the needle, and a second front transverse branch (17b) parallel to the first branch, located in the said chamber and equipped with a perforation (20) for the passage of the needle, where the perforation (19) of the rear branch is not able to allow the passage of the modified section of the needle, but the perforation (20) of the front branch is able to allow this section to pass.

6. A device according to claim 5, in which the catheter base has an external rim (13) and the cage includes a mobile lever (26) which has a stop dog (14) locked to this rim in one position of the lever, and in which the second branch (17b) of the flexible blade is continued by a third branch (17c) substantially at the bracket of the second branch, and which operates the said lever to release the dog.

7. A device according to one of claims 1 to 6, in which, ahead of the chamber, the cage includes a nose (11) which slots into the catheter base and which is traversed longitudinally by an aperture (12) for the passage of the needle.

8. A device according to any one of claims 1 to 7, in which the needle (3) is equipped with a base (4), and in which the cage includes a transverse plate (24) projecting laterally, against which presses one wall (25) of the base of the needle when the needle is in its working position.

9. A device according to claim 8, in which the said blade (12) is suspended from the said plate (24) of the cage.

10. A device according to any one of claims 1 to 9, in which the needle (3) includes a base (4) bearing against the flexible blade when the needle is in its working position.

## Patentansprüche

1. Vorrichtung zum Einführen in den Körper, durch die Haut hindurch, mit einem Katheter (1) an einem proximalen Ansatz (2), wobei die Vorrichtung eine Nadel (3) umfasst, die ein Punktionsende aufweist, sowie einen Käfig (5), der den Ansatz in proximaler Richtung verlängert, wobei dieser Käfig eine Kammer (6) festlegt, die gleitend von einem proximalen Eingang (7) zu einem gegenüberliegenden distalen Ausgang (9) von der Nadel durchquert wird und mit einer flexiblen Zunge aus Federstahl (17; 17b) versehen ist, um in der Kammer das Punktionsende der Nadel zurückzuhalten, wenn die Nadel aus der Kanüle gezogen wird, **dadurch gekennzeichnet, dass** diese Zunge quer zur Kammer nahe des proximalen Eingangs (7) der Kammer senkrecht zur Nadel angeordnet ist und von der Nadel durchquert wird, wobei die Zunge und die Nadel Mittel (19; 21; 22) umfassen, die zusammenwirken, damit die Zunge im Ruhezustand ist und frei von der Nadel durchquert wird, wenn die Nadel in distaler Richtung gestoßen wird und damit die Zunge die Nadel aufhält und durch die Nadel gebogen wird, wenn die Nadel in proximaler Richtung jenseits einer bestimmten axialen Position gezogen wird, damit die gebogene Zunge die Nadel neigt und auf die geneigte Nadel eine Rückstellkraft ausübt, die danach strebt, die Nadel in distaler Richtung bis zum Anschlag des Punktionsende gegen eine Wand der Kammer zurückzustoßen.

2. Vorrichtung gemäß Anspruch 1, in der die flexible Zunge eine Perforierung (19) für den Durchgang der Nadel aufweist und die Nadel lokal vor der Perforierung einen veränderten Nadelabschnitt (21;22) aufweist, damit dieser Abschnitt durch die Perforierung der Zunge bei der Rückzugsbewegung der Nadel in proximaler Richtung aufgehalten wird, wobei dieser veränderte Abschnitt in einem Punktionsabstand der Nadel ist, so dass der Kontakt des veränderten Abschnitts mit der Perforierung der Zunge entsteht, nachdem dieses Ende in der Kammer angekommen ist, bei der Rückzugsbewegung der Nadel.

3. Vorrichtung gemäß Anspruch 1 oder 2, in der die Kammer eine Bodenwand (8) in distaler Richtung aufweist, die eine Rille (10) aufweist, in die sich das Punktionsende (3a) der geneigten Nadel anordnet.

4. Vorrichtung gemäß einem der Ansprüche 1 bis 3, in der die flexible Zunge einen Arm (17) einer in L-Form ausgestalteten Zunge bildet und die einen an einer Längswand der Kammer befestigten Längsarm (16) aufweist, sowie einen Querarm (17), der die flexible Zunge bildet, die mit einer Perforierung (19) für den Durchgang der Nadel ausgestattet ist.

5. Vorrichtung gemäß einem der Ansprüche 1 bis 3, in der die flexible Zunge in U-Form ausgestaltet ist, wobei sie einen ersten hinteren Querarm (17a) bildet, der am Eingang der Kammer gelegen ist und mit einer Perforierung (19) für den Durchgang der Nadel ausgestattet ist, sowie einen zweiten vorderen Querarm (17b), parallel zum ersten Arm, der in der Kammer gelegen ist und mit einer Perforierung (20) für den Durchgang der Nadel ausgestattet ist, wobei die Perforierung (19) des hinteren Arms nicht ausgelegt ist, den veränderten Abschnitt der Nadel durchgehen zu lassen, sondern die Perforierung (20) des vorderen Arms ausgelegt ist, diesen Abschnitt durchgehen zu lassen.

6. Vorrichtung gemäß Anspruch 5, in welcher der Ansatz des Katheters eine äußere Kante (13) aufweist und der Käfig einen beweglichen Hebel (26) trägt, der ein Haltestück (14) in Eingriff mit dieser Kante in einer Position des Hebels aufweist, und in welcher der zweite Arm (17b) der flexiblen Zunge durch einen dritten Arm (17c) im wesentlichen im Winkel des zweiten Arms fortgesetzt wird, und der den Hebel betätigt, um den Eingriff aufzuheben.

7. Vorrichtung gemäß einem der Ansprüche 1 bis 6, in welcher der Käfig vor der Kammer eine Nase (11) umfasst, die in den Ansatz des Katheters passt und die der Länge nach von einer Öffnung (12) für den Durchgang der Nadel durchquert wird.

8. Vorrichtung gemäß einem der Ansprüche 1 bis 7, in der die Nadel (3) mit einem Ansatz (4) versehen ist und in welcher der Käfig eine Querplatte (24) im seitlichen Vorsprung umfasst, gegen die eine Wand (25) des Ansatzes der Nadel in Anschlag kommt, wenn die Nadel in Betriebsposition ist.

9. Vorrichtung gemäß Anspruch 8, in der die Zunge (12) an der Platte (24) des Käfigs aufgehängt ist.

10. Vorrichtung gemäß einem der Ansprüche 1 bis 9, in der die Nadel (3) einen Ansatz (4) in Anschlag gegen die flexible Zunge umfasst, wenn die Nadel in Betriebsposition ist.
